# FASCICULE DE BREVET EUROPEEN

(11) **EP 3 164 201 B1**
(45) Date de publication et mention de la délivrance du brevet: **07.05.2025**
(21) Numéro de dépôt: 15734366.6
(22) Date de dépôt: 06.07.2015
(51) Int. Cl.: B01D 9/00, B01J 2/04, C06B 21/00, B01D 1/16

(54) **PROCEDE DE PREPARATION DE CO-CRISTAUX PAR EVAPORATION FLASH**
VERFAHREN ZUR HERSTELLUNG VON CO-KRISTALLEN MITTELS ENTSPANNUNGSVERDAMPFUNG
METHOD FOR PRODUCING COCRYSTALS BY MEANS OF FLASH EVAPORATION

(30) Priorité: 04.07.2014 FR 1456461
(43) Date de publication de la demande: 10.05.2017
(73) Titulaire: Centre National de la Recherche Scientifique (C.N.R.S.), 75016 Paris (FR); Université de Strasbourg, 67000 Strasbourg (FR); ISL - Institut franco-allemand de recherches de Saint-Louis, 68300 Saint-Louis (FR)
(72) Inventeur: RISSE, Benedikt, 85560 Ebersberg (DE); SPITZER, Denis, 67203 Oberschaeffolsheim (FR); PESSINA, Florent, 67000 Strasbourg (FR)
(74) Mandataire: Brevalex
(86) Numéro de dépôt international: PCT/EP2015/065335
(87) Numéro de publication internationale: WO 2016/001445

(56) Documents cités:
- WO-A1-2010/007446
- WO-A1-2013/117671
- WO-A1-2013/117671
- US-A- 3 833 479
- US-A- 4 795 330
- US-A1- 2008 280 858
- US-A1- 2011 250 264

## Description

L'invention concerne un procédé de préparation d'un co-cristal d'au moins deux composés par évaporation instantanée ou évaporation flash, par exemple pour la préparation de co-cristaux dans les domaines des matériaux énergétiques, des composés pharmaceutiques, des composés phytopharmaceutiques, des matériaux ferroélectriques, des matériaux à réponse non-linéaire ou des matériaux bio-électroniques.

Les co-cristaux sont des solides assemblés à l'échelle moléculaire. Plusieurs types d'interactions intermoléculaires peuvent permettre la préparation de co-cristaux. Ces interactions peuvent être des liaisons hydrogène, des liaisons ioniques, des liaisons de type empilement (π stacking) ou encore des liaisons de Van der Walls.

Les co-cristaux sont généralement plus stables thermodynamiquement que les composés de départ.

Les co-cristaux possèdent généralement des propriétés améliorées par rapport aux composés correspondant utilisés individuellement.

Ainsi, les co-cristaux de substances pharmaceutiques ou phytopharmaceutiques, de matériaux énergétiques ou de matériaux ferroélectriques possèdent des propriétés améliorées.

Par exemple, les co-cristaux de substances pharmaceutiques ou phytopharmaceutiques possèdent généralement une meilleure solubilité et donc une meilleure biodisponibilité. Ils possèdent également une stabilité améliorée, notamment en conditions humides.

De même, les co-cristaux de matériaux énergétiques possèdent des propriétés améliorées, notamment une meilleure réactivité combinée à une sensibilité réduite, propriété essentielle lors de leur manipulation.

Toutefois, les procédés de préparation de co-cristaux de l'état de la technique présentent des inconvénients limitant fortement le développement de l'utilisation de co-cristaux.

Ainsi, les procédés de l'état de la technique ne sont pas des procédés continus ou semi-continus mais des procédés de préparation par lots ou procédés batch. Ces procédés de l'état de la technique ne permettent donc pas des rendements élevés.

Quatre types de procédés de l'état de la technique permettent de préparer des co-cristaux. On connait la cristallisation par évaporation lente d'une solution concentrée de plusieurs composés, le broyage des réactifs à l'état solide éventuellement en présence d'un solvant, les réactions induites électro-chimiquement et la cristallisation à cinétique contrôlée par évaporation rapide du solvant d'une solution des composés purs.

Toutefois, ces procédés ne permettent pas la préparation continue ou semi-continue de co-cristaux.

De plus, les co-cristaux préparés selon les procédés connus ne sont pas toujours d'une qualité suffisante.

La taille moyenne des co-cristaux préparés selon les procédés connus n'est pas toujours régulière et n'est pas systématiquement micrométrique, submicrométrique ou nanométrique. De plus, les procédés connus ne permettent pas d'atteindre des vitesses de cristallisation élevées.

Par ailleurs, on connaît la technologie RESS (Rapid Expansion of Supercritical Solutions) pour la préparation de nanoparticules dans un fluide supercritique. Cette technologie n'est efficace qu'à échelle réduite et ne peut donc être transférée au niveau industriel. De plus, la préparation dans un fluide supercritique ne permet pas de contrôler la stœchiométrie. On connait également par la demande WO 2013-117671 une méthode de préparation de nanoparticules d'un matériau composite. Le document WO 2010/007446 A1 décrit un procédé visant à augmenter la cristallinité d'un matériau solide comprenant un composé fluticasone par application d'ultrasons. Le document US 4,795,330 A décrit un procédé de production de particules solides par solidification en vol d'un jet chargé électriquement de gouttelettes de fluide de distribution granulométrique étroite ainsi qu'un appareil pour la mise en œuvre de ce procédé. Toutefois, aucun de ces procédés ne permet de préparer des co-cristaux.

Il existe donc un besoin de disposer d'un procédé de préparation de co-cristaux apportant une solution aux problèmes des procédés de préparation de co-cristaux de l'état de la technique.

Ainsi, l'invention fournit un procédé de préparation d'un co-cristal d'au moins deux composés par évaporation instantanée ou évaporation flash qui permet d'apporter une solution à tout ou partie des problèmes des procédés de l'état de la technique.

L'invention concerne un procédé de préparation d'un co-cristal d'au moins deux composés liés par des liaisons hydrogène, des liaisons ioniques, des liaisons de type empilement (π-π stacking) ou des liaisons de Van der Walls, ce procédé comprenant les étapes successives :
▪ préparation d'au moins deux solutions comprenant chacune au moins un solvant et au moins un composé, organique, minéral ou organométallique, ces composés pouvant se lier par liaisons hydrogène, par liaisons ioniques, par liaisons de type empilement (π- π stacking) ou par liaisons de Van der Walls ;
▪ chauffage des solutions, sous une pression allant de 3 à 300 bar, à une température supérieure au point d'ébullition du solvant ou à une température supérieure au point d'ébullition du mélange de solvants ;
▪ atomisation dans une même chambre d'atomisation de chaque solution au moyen d'au moins un dispositif de dispersion et sous un angle allant de 30 à 150 ° à une pression allant de 0,0001 à 2 bar, les dispositifs de dispersion étant deux buses à cône creux installées côte à côte dans la chambre d'atomisation, chacune équipée d'un système de chauffage électrique, et orientées l'une par rapport à l'autre de manière à ce que leurs jets s'interpénètrent ;
▪ séparation des solvants sous forme gazeuse.

Le procédé selon l'invention est avantageusement mis en œuvre de manière continue ou de manière semi-continue. De préférence, il est mis en œuvre de manière continue.

De manière avantageuse, le procédé selon l'invention comprend la préparation d'au moins une solution comprenant
▪ deux à dix composés ; ou
▪ deux composés ; ou
▪ deux composés en un ratio molaire choisi parmi 1/4, 1/3, 1/2, 1/1, 2/1, 3/1, 4/1 ; ou
▪ trois composés ; ou
▪ trois composés en un ratio molaire X/Y/Z dans lequel X, Y et Z, identiques ou différents, représentent 1, 2, 3 ou 4 ;
▪ quatre composés ; ou
▪ quatre composés en un ratio molaire W/X/Y/Z dans lequel W, X, Y et Z, identiques ou différents, représentent 1, 2, 3 ou 4 ;
▪ cinq composés ; ou
▪ cinq composés en un ratio molaire V/W/X/Y/Z dans lequel V, W, X, Y et Z, identiques ou différents, représentent 1, 2, 3 ou 4.

De manière préférée, le procédé selon l'invention comprend la préparation d'au moins une solution comprenant deux, trois ou quatre composés.

De manière également préférée, le procédé selon l'invention comprend la préparation d'au moins deux solutions comprenant chacune au moins un solvant et au moins un composé, organique, minéral ou organométallique, ces composés, identiques ou différents, pouvant se lier par liaisons hydrogène, par liaisons ioniques, par liaisons de type empilement (π- π stacking) ou par liaisons de Van der Walls. Ces solutions peuvent chacune indépendamment comprendre plusieurs de ces composés.

Le procédé selon l'invention est particulièrement avantageux pour la préparation de co-cristaux de composés choisis parmi les composés énergétiques, les composés pharmaceutiques, les composés phytopharmaceutiques, les composés colorants, les pigments, les encres, les peintures, les oxydes métalliques.

De préférence, le procédé selon l'invention est mis en œuvre pour la préparation de co-cristaux de composés choisis parmi les composés énergétiques, les composés pharmaceutiques, les composés phytopharmaceutiques.

De manière également avantageuse, le procédé selon l'invention permet de préparer des co-cristaux dont la taille est micrométrique ou qui possèdent au moins une dimension inférieure à 500 µm, de préférence qui possèdent au moins une dimension inférieure à 100 µm.

De manière également avantageuse, le procédé selon l'invention permet de préparer des co-cristaux dont la taille est submicrométrique ou qui possèdent au moins une dimension comprise entre 100 et 1 000 nm.

De manière préférée, le procédé selon l'invention permet de préparer des co-cristaux dont la taille est nanométrique ou qui possèdent au moins une dimension inférieure à 100 nm.

De manière plus préférée, les co-cristaux préparés selon l'invention ont une taille allant de 2 à 100 nm ; ou allant de 5 à 90 nm ; ou allant de 10 à 80 nm ; ou allant de 50 à 300 nm ; ou allant de 50 à 200 nm ; ou allant de 50 à 120 nm ; ou allant de 10 à 100 nm ; ou allant de 60 à 100 nm.

De manière avantageuse, le procédé selon l'invention comprend la préparation d'au moins une solution comprenant au moins deux composés organiques, minéraux ou organométalliques et au moins deux solvants.

De manière également avantageuse, le procédé selon l'invention comprend la préparation d'au moins une solution comprenant au moins un solvant d'un des composés et au moins un co-solvant d'un des composés.

De manière également avantageuse, le procédé selon l'invention comprend la préparation d'au moins une solution comprenant au moins un solvant d'un des composés et au moins un anti-solvant d'un des composés.

De manière préférée, le ou les solvants mis en œuvre ont un point d'ébullition inférieur à 80 °C ou inférieur à 60 °C. Comme solvant, on peut citer les alcanes, par exemple le pentane (PE= 36 °C) ou l'hexane (PE= 68 °C) ; les alcools, par exemple le méthanol (PE= 65 °C) ou l'éthanol (PE= 78-79 °C) ; les thiols, par exemple l'éthane-thiol (PE= 35 °C) ; les aldéhydes, par exemple l'éthanal (PE= 20 °C) ou l'aldéhyde propionique (PE= 48 °C) ; les cétones, par exemple l'acétone (PE= 56 °C) ; les éthers, par exemple le méthyl-tert-butyl éther (PE= 55 °C) ou le tetrahydrofurane (PE= 66 °C) ; les esters d'acides, notamment les esters d'acide formique, par exemple le formiate de méthyle (PE= 32 °C), les esters d'acide acétique, par exemple l'acétate de méthyle (PE= 57-58 °C) ; les amines, par exemple la triméthylamine (PE= 2-3 °C).

De manière préférée, le procédé selon l'invention comprend une étape finale de récupération des co-cristaux de composés.

De manière plus préférée, la récupération des co-cristaux de composés est réalisée au moyen d'un ou plusieurs dispositifs choisis parmi un séparateur électrostatique, un cyclone, un cyclone comprenant un dispositif électrostatique.

Les conditions de mise en œuvre du procédé selon l'invention peuvent varier assez largement, notamment en fonction des composés à co-cristalliser ou bien en fonction des solvants utilisés.

De manière avantageuse, le chauffage respectif de chaque solution peut être effectué sous une pression allant de 5 à 150 bar ou allant de 10 à 60 bar qui peut être identique ou différente pour chaque solution.

De manière également avantageuse, le chauffage des solutions est effectué sous pression d'un gaz inerte choisi parmi l'azote, l'argon, l'hélium, le néon, le xénon.

Lors de l'atomisation des solutions, la pression est avantageusement comprise entre 0,001 et 2 bar.

Les dispositifs de dispersion mis en œuvre lors de l'atomisation des solutions sont des buses à cône creux.

L'invention concerne également un dispositif permettant la mise en œuvre du procédé décrit ci-dessus. Ainsi, l'invention fournit un dispositif permettant la préparation d'un co-cristal d'au moins deux composés pouvant se lier par liaisons hydrogène, par liaisons ioniques, par liaisons de type empilement (π- π stacking) ou par liaisons de Van der Walls, ce dispositif comprenant
▪ au moins deux réacteurs comprenant chacun
   - une alimentation en une solution de chaque composé et d'au moins un solvant ;
   - au moins un réservoir de stockage sous forte pression de ladite solution de chaque composé ;
   - au moins un dispositif de mise sous pression pouvant aller de 3 à 300 bar ;
   - au moins un dispositif de chauffage de la solution à une température supérieure au point d'ébullition du solvant ou à une température supérieure au point d'ébullition du mélange de solvants ;
▪ une chambre d'atomisation comprenant
   - au moins un dispositif de dispersion de la solution sous un angle allant de 30 à 150° et à une pression allant de 0,0001 à 2 bar, les dispositifs de dispersion étant deux buses à cône creux (3) installées côte à côte dans la chambre d'atomisation, chacune équipée d'un système de chauffage électrique, et orientées l'une par rapport à l'autre de manière à ce que leurs jets s'interpénètrent ;
   - au moins un dispositif de séparation de solvant ;
▪ un ou plusieurs dispositifs de récupération des nanoparticules de composés choisis parmi un séparateur électrostatique, un cyclone, un cyclone comprenant un dispositif électrostatique.

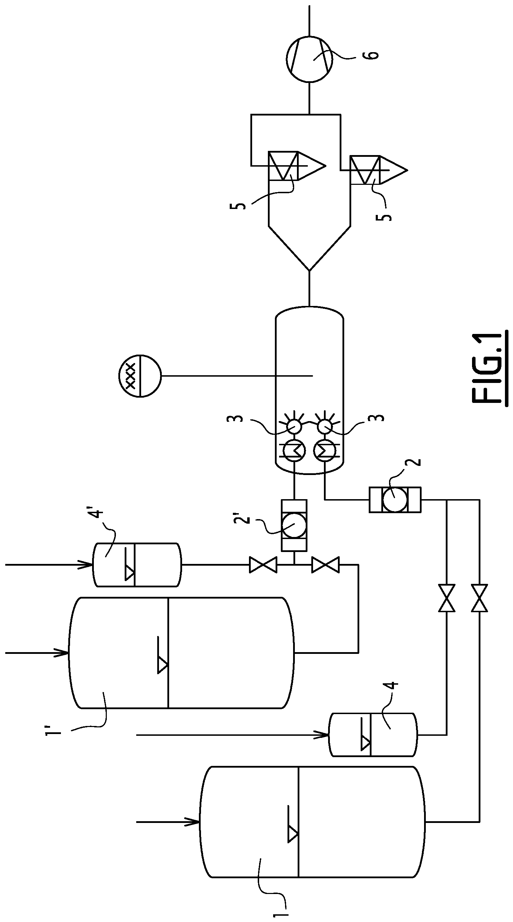

Un mode de mise en œuvre d'un dispositif selon l'invention est représenté par la figure 1. Le dispositif est composé de quatre parties principales : un ensemble de deux réservoirs (1 et 1') pour le stockage sous forte pression des fluides contenant la ou les substances à cristalliser, une chambre d'atomisation comprenant deux buses chauffées (3) intégrées, deux cyclones axiaux (5) montés en parallèle et permettant une production semi-continue, une pompe à vide (6).

Dans les réservoirs (1 et 1') de 5 L contenant le solvant avec le soluté, on applique une surpression d'azote comprimé. Dans un premier temps, cette surpression permet de déplacer l'oxygène et empêche l'évaporation du solvant. Le débit volumique dans ce système est induit par la surpression d'azote comprimé.

Des filtres (2 et 2') de 15 µm refoulent toutes les impuretés solides dans la solution initiale.

Deux buses à cône creux (3), chacune équipée d'un système de chauffage électrique, sont installées côte à côte dans la chambre d'atomisation. On contrôle les paramètres de pression, de température et de distribution de la taille des particules. Le type de branchement permet un changement rapide des buses. La température du chauffage électrique est choisie par l'utilisateur et régulée automatiquement. Les buses sont orientées l'une par rapport à l'autre de manière à ce que leurs jets s'interpénètrent.

Un réservoir ou bac de solvant (4) est rempli avec le même solvant que le réservoir (1) et sert à rincer la conduite et la buse après utilisation. De même, le réservoir ou bac de solvant (4') est rempli avec le même solvant que le réservoir (1').

Les cyclones axiaux (5) sont installés en parallèle. Pendant l'opération, seul un cyclone est en service ; le deuxième cyclone est en veille. Grâce à la force centrifuge, les particules solides se déposent à l'intérieur du cyclone, les composants gazeux quittent le cyclone par un tuyau plongeur. Pour vider le cyclone, on ouvre d'abord le circuit conduisant vers le second cyclone, pour ensuite fermer le premier circuit conduisant vers le premier cyclone.

La pompe à vide (6) assure un écoulement permanent dans l'installation et permet d'extraire les vapeurs de solvant du système.

Les différents aspects de l'invention sont illustrés par l'exemple 2 qui suit.

### Exemple 1 : préparation de co-cristaux à partir d'une solution (hors invention)

Des co-cristaux ont été préparés à partir de caféine et d'acide oxalique ou d'acide glutarique. D'autres co-cristaux ont été préparés à partir de 2,4,6,8,10,12-hexanitro-2,4,6,8,10,12-hexaazaiso-wurtzitane (CL-20) et de 2,4,6-trinitrotoluene (TNT) ou de 1,3,5,7-tetranitro-1,3,5,7-tetrazacyclooctane (HMX).

Des exemples comparatifs ont été préparés à partir de de 2,4,6-trinitrotoluene (TNT) et de 1,3,5,7-tetranitro-1,3,5,7-tetrazacyclooctane (HMX).

Les co-cristaux ont été préparés de manière continue au moyen du dispositif décrit dans la demande de brevet internationale WO-2013/117671 selon un procédé d'évaporation instantanée d'une solution des composés à co-cristalliser qui est surchauffée et comprimée. Au cours du procédé, la solution subit une très forte chute de la pression au moment d'être atomisée au moyen d'une buse à cône creux.

Les composés à co-cristalliser sont dissous dans un solvant dont le point d'ébullition est généralement inférieur à 60 °C. Les composés et les solvants ainsi que les paramètres de réaction mis en œuvre sont présentés dans le tableau 1.

La solution est comprimée (40 à 60 bar) puis atomisée dans une chambre d'atomisation au moyen d'une buse à cône creux chauffée.

La pression dans la chambre d'atomisation (5 mbar) est obtenue au moyen d'une pompe à vide (35 m³/h).

La chute de pression soudaine entraîne un déplacement de l'équilibre thermodynamique rendant instable la solution surchauffée. Le solvant est évaporé instantanément et les co-cristaux se forment.

La forte chute de la pression s'accompagne d'une forte baisse de la température qui baisse d'environ 200 °C permettant de protéger les co-cristaux formés.

La séparation continue des co-cristaux formés est réalisée au moyen de cyclones axiaux montés en parallèle.

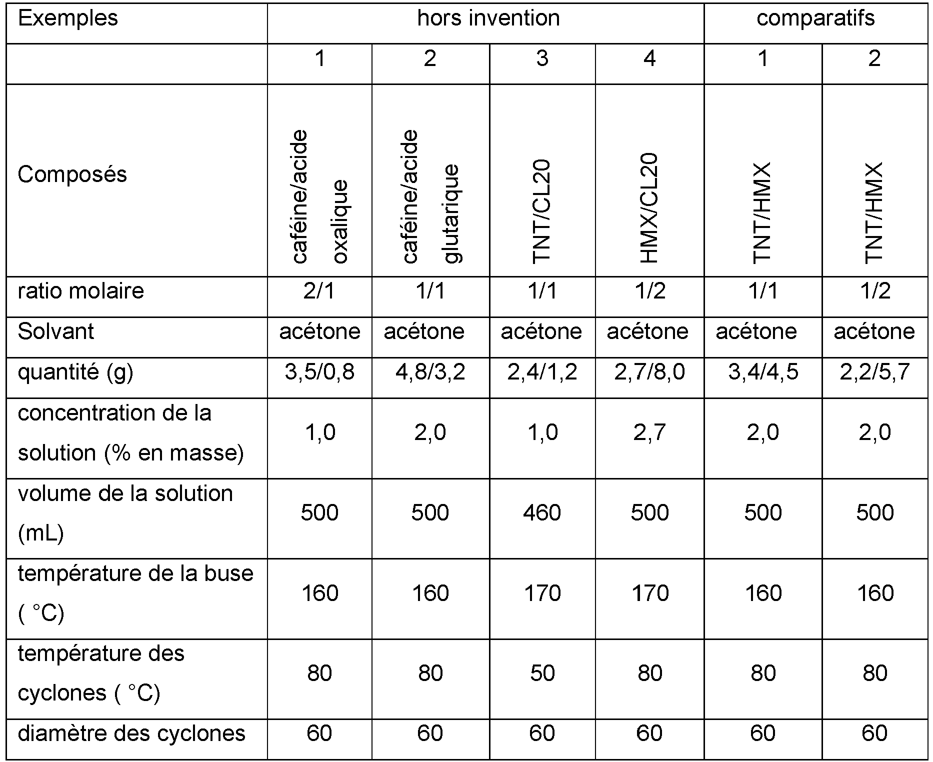

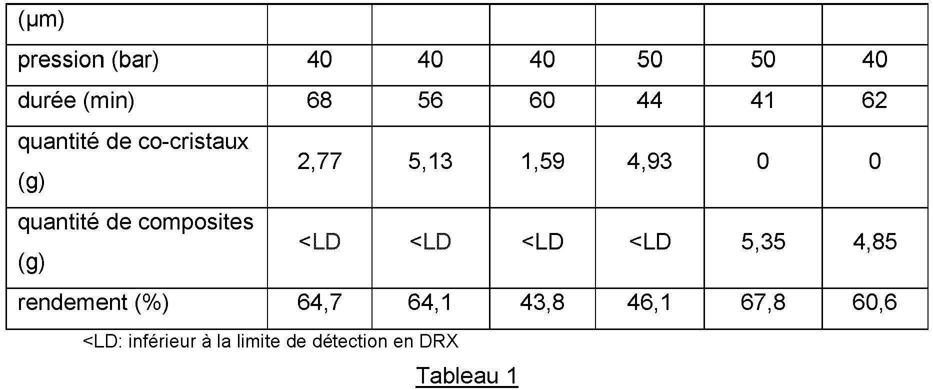

Les produits ont été caractérisés par microscopie AFM (Atomic Force Microscopy) à température ambiante et à pression atmosphérique afin de ne pas altérer les co-cristaux formés.

La distribution de taille moyenne des particules a été évaluée. La taille moyenne des co-cristaux caféine/acide glutarique (1/1) est de 111 nm. La taille moyenne des co-cristaux HMX/CL20 (1/2) est de 59 nm.

Des spectres de diffraction de rayons X ont été réalisés pour caractériser les co-cristaux.

Les spectres obtenus ont été comparés aux spectres des produits de départ utilisés. Les spectres des co-cristaux sont différents des spectres des produits de départ pour les co-cristaux formés. Ils correspondent aux spectres de la banque de données structurales de Cambridge ou aux spectres disponibles dans la littérature.

Pour les composites TNT/HMX (1/1 et 1/2), les spectres de diffraction de rayons X présentent toujours les raies caractéristiques du TNT seul ainsi que certaines raies du HMX. L'absence de certaines raies du HMX indique que HMX est présent sous une forme amorphe. Les composites TNT/HMX (1/1 et 1/2) sont donc des mélanges de cristaux de TNT et de HMX amorphe.

Les propriétés thermiques des co-cristaux ont été étudiées par DSC (Differential Scanning Calorimetry).

Le chauffage (5 °C/min) des co-cristaux TNT/CL20 (1/1) montre l'absence du signal de fusion caractéristique du TNT pur à 80 °C, le TNT étant au sein de la maille du co-cristal avec le CL20. La température de fusion caractéristique mesurée pour le co-cristal TNT/CL20 (1/1) est de 135 °C.

Après la première phase de l'analyse DSC, la température est réduite puis à nouveau augmentée. Lors du deuxième chauffage, le signal thermique du TNT est à nouveau présent à 80 °C confirmant la dissociation du co-cristal sous l'action de la chaleur lors de la première chauffe suivie de la cristallisation du TNT pur.

Le chauffage des co-cristaux caféine/acide oxalique (2/1) montre la présence d'un signal thermique à 199 °C qui est intermédiaire entre les points de fusion des deux composés caféine et acide oxalique purs.

La poursuite du chauffage conduit à la dissociation du co-cristal. Puis le refroidissement suivi du deuxième chauffage montre la présence du signal thermique de la caféine pure.

Pour les composites TNT/HMX (1/1 et 1/2) des exemples comparatifs, le signal thermique du TNT est présent dès le premier chauffage. Puis ce signal n'est pas modifié lors du deuxième chauffage. Les composites TNT/HMX (1/1 et 1/2) sont donc de simples mélanges physiques de particules de TNT et de HMX. Les molécules de TNT et de HMX ne peuvent pas former de liaisons intermoléculaires pour donner un co-cristal.

### Exemple 2 : préparation de co-cristaux à partir de deux solutions (selon l'invention)

Des co-cristaux selon l'invention ont été préparés de manière continue à partir de HMX et de CL20 au moyen du dispositif de la figure 1.

On dissout 5 g de CL20 préalablement séché dans 250 ml d'acétone (CHROMASOLV^{®} qualité HPLC ≥99.9% de Sigma Aldrich). Par ailleurs, on dissout 1,35 g de HMX préalablement séché dans 250 ml d'acétone (CHROMASOLV^{®} qualité HPLC ≥99.9% de Sigma Aldrich).

Chaque solution est mise à agiter puis passée aux ultrasons pendant 10 secondes. Ensuite, chaque solution est versée dans un réservoir de 1 L : la solution d'acétone avec CL20 dans le réservoir 1 et la solution d'acétone avec HMX dans le réservoir 1'. À chaque réservoir 1 et 1' est connecté en parallèle un réservoir d'acétone technique, indiqué par les numéros 2 et 2'. Tous les réservoirs sont fermés et mis sous pressions à 40 bar par injection d'azote sous pression. La pression est mesurée avant la buse et contrôlée tout au long de la réaction.

Le vide est fait dans tout le système par la mise en route de la pompe. Une fois le vide stabilisé à environ 0,1 mbar un des deux cyclones est isolé du système.

Les vannes reliant les réservoirs 2 et 2' sont ouvertes et les systèmes de chauffage sont mis en route et réglés pour les buses à 170 °C et pour les cyclones à 80 °C. Une fois les températures stabilisées, le cyclone utilisé est isolé et l'autre cyclone isolé est ouvert. Puis les solutions des réservoirs 1 et 1' sont pulvérisées.

Après 20 minutes, les cyclones sont de nouveaux inversés puis les vannes alimentant les buses basculées sur les réservoirs 2 et 2' d'acétone technique. Les systèmes de chauffage sont ensuite arrêtés.

Pendant le refroidissement, le co-cristal CL20-HMX est récupéré dans le cyclone ayant servi lorsque les solutions 1 et 1' étaient pulvérisées. Une fois les températures inférieures à 50 °C, les arrivées d'acétone technique sont coupées et la pompe à vide est arrêtée après avoir permis à la pression de remonter. On forme ainsi *in situ* du cocrystal CL20-HMX 2:1 à 2,67 % en poids en solution par évaporation instantanée ou évaporation flash multi-buses.

Le produit a été caractérisé par microscopie AFM (Atomic Force Microscopy) à température ambiante et à pression atmosphérique afin de ne pas altérer le co-cristal formé.

La distribution de taille moyenne des particules a été évaluée. La taille moyenne du co-cristal CL20-HMX (2/1) est de 60 nm. Un spectre de diffraction de rayons X a été réalisé pour caractériser le co-cristal. Le spectre obtenu a été comparé aux spectres des produits de départ utilisés et à celui du co-cristal obtenu dans l'exemple 1. Le spectre présente toutes les raies caractéristiques du co-cristal CL20-HMX (2/1) que ce soit comparé à la littérature ou au co-cristal CL20-HMX (2/1) obtenu dans l'exemple 1. Le spectre présente aussi les raies caractéristiques de la phase beta du CL20.

## Revendications

1. Procédé de préparation d'un co-cristal d'au moins deux composés liés par des liaisons hydrogène, des liaisons ioniques, des liaisons de type empilement (π-π stacking) ou des liaisons de Van der Walls, comprenant les étapes successives :
▪ préparation d'au moins deux solutions comprenant chacune au moins un solvant et au moins un composé, organique, minéral ou organométallique, ces composés pouvant se lier par liaisons hydrogène, par liaisons ioniques, par liaisons de type empilement (π-π stacking) ou par liaisons de Van der Walls ;
▪ chauffage des solutions, sous une pression allant de 3 à 300 bar, à une température supérieure au point d'ébullition du solvant ou à une température supérieure au point d'ébullition du mélange de solvants ;
▪ atomisation dans une même chambre d'atomisation de chaque solution au moyen d'au moins un dispositif de dispersion et sous un angle allant de 30 à 150 ° à une pression allant de 0,0001 à 2 bar, les dispositifs de dispersion étant deux buses à cône creux (3) installées côte à côte dans la chambre d'atomisation, chacune équipée d'un système de chauffage électrique, et orientées l'une par rapport à l'autre de manière à ce que leurs jets s'interpénètrent ;
▪ séparation des solvants sous forme gazeuse.

2. Procédé selon la revendication 1, dans lequel lesdites au moins deux solutions sont chauffées dans au moins deux réacteurs, lesdits réacteurs comprenant une alimentation en une solution de chaque composé, au moins un dispositif de mise sous pression pouvant aller de 3 à 300 bar et au moins un dispositif de chauffage, et dans lequel lesdites au moins deux solutions sont atomisées dans ladite même chambre d'atomisation.

3. Procédé selon la revendication 1 ou 2 comprenant la préparation d'au moins une solution comprenant
▪ deux à dix composés ; ou
▪ deux composés ; ou
▪ deux composés en un ratio molaire choisi parmi 1/4, 1/3, 1/2, 1/1, 2/1, 3/1, 4/1 ; ou
▪ trois composés ; ou
▪ trois composés en un ratio molaire X/Y/Z dans lequel X, Y et Z, identiques ou différents, représentent 1, 2, 3 ou 4 ;
▪ quatre composés ; ou
▪ quatre composés en un ratio molaire W/X/Y/Z dans lequel W, X, Y et Z, identiques ou différents, représentent 1, 2, 3 ou 4 ;
▪ cinq composés ; ou
▪ cinq composés en un ratio molaire V/W/X/Y/Z dans lequel V, W, X, Y et Z, identiques ou différents, représentent 1, 2, 3 ou 4.

4. Procédé selon l'une des revendications 1 à 3 pour lequel le co-cristal
▪ est de taille micrométrique ; ou
▪ possède au moins une dimension inférieure à 500 µm ; ou
▪ possède au moins une dimension inférieure à 100 µm ; ou
▪ est de taille submicrométrique ; ou
▪ possède au moins une dimension comprise entre 100 nm et 1 000 nm ; ou
▪ est de taille nanométrique ; ou
▪ possède au moins une dimension inférieure à 100 nm ; ou
▪ est de taille allant de 2 à 100 nm ; ou
▪ est de taille allant de 5 à 90 nm ; ou
▪ est de taille allant de 10 à 80 nm ; ou
▪ est de taille allant de 50 à 300 nm ; ou
▪ est de taille allant de 50 à 200 nm ; ou
▪ est de taille allant de 50 à 120 nm ; ou
▪ est de taille allant de 10 à 100 nm ; ou
▪ est de taille allant de 60 à 100 nm.

5. Procédé selon l'une des revendications 1 à 4 comprenant la préparation d'au moins une solution comprenant au moins deux composés organiques, minéraux ou organométalliques et au moins deux solvants ou au moins un solvant et au moins un co-solvant ou au moins un solvant et au moins un anti-solvant d'un des composés.

6. Procédé selon l'une des revendications 1 à 5 comprenant une étape finale de récupération des co-cristaux de composés au moyen d'un ou plusieurs dispositifs choisis parmi un séparateur électrostatique, un cyclone (5), un cyclone comprenant un dispositif électrostatique.

7. Procédé selon l'une des revendications 1 à 6 qui est continu ou semi-continu.

8. Procédé selon l'une des revendications 1 à 7 pour lequel le point d'ébullition du solvant ou du mélange de solvants est inférieur à 80 °C ou est inférieur à 60 °C.

9. Procédé selon l'une des revendications 1 à 7 pour lequel le chauffage des solutions est effectué sous une pression allant de 5 à 150 bar ou allant de 10 à 60 bar.

10. Procédé selon l'une des revendications 1 à 9 pour lequel le chauffage des solutions est effectué sous pression d'un gaz inerte choisi parmi l'azote, l'argon, l'hélium, le néon, le xénon.

11. Procédé selon l'une des revendications 1 à 9 pour lequel l'atomisation des solutions est réalisée à une pression allant de 0,001 à 2 bar.

12. Procédé selon l'une des revendications 1 à 11 pour lequel les composés sont choisis parmi les composés énergétiques, les composés pharmaceutiques, les composés phytopharmaceutiques, les composés colorants, les pigments, les encres, les peintures, les oxydes métalliques.

13. Procédé selon l'une des revendications 1 à 12 pour lequel le solvant est choisi parmi les alcanes, par exemple le pentane (PE= 36 °C) ou l'hexane (PE= 68 °C) ; les alcools, par exemple le méthanol (PE= 65 °C) ou l'éthanol (PE= 78-79 °C) ; les thiols, par exemple l'éthane-thiol (PE= 35 °C) ; les aldéhydes, par exemple l'éthanal (PE= 20°C) ou l'aldéhyde propionique (PE= 48 °C) ; les cétones, par exemple l'acétone (PE= 56 °C) ; les éthers, par exemple le méthyl-tert-butyl éther (PE= 55 °C) ou le tetrahydrofurane (PE= 66 °C) ; les esters d'acides, notamment les esters d'acide formique, par exemple le formiate de méthyle (PE= 32 °C), les esters d'acide acétique, par exemple l'acétate de méthyle (PE= 57-58 °C) ; les amines, par exemple la triméthylamine (PE= 2-3 °C).

14. Procédé selon l'une des revendications 1 à 13, **caractérisé en ce que** lesdites solutions sont stockées dans un ensemble de deux réservoirs (1, 1') de stockage sous forte pression, lesdites solutions sont atomisées dans la chambre d'atomisation comprenant deux buses à cône creux (3) chauffées intégrées, et lesdits co-cristaux sont récupérés dans deux cyclones axiaux (5) montés en parallèle et permettant une production semi-continue, et dans lequel une pompe à vide (6) assure un écoulement permanent dans l'installation et permet d'extraire les vapeurs de solvant.

15. Dispositif permettant la mise en œuvre d'un procédé selon les revendications 1 à 14 comprenant
▪ au moins deux réacteurs comprenant chacun
- une alimentation en une solution de chaque composé et d'au moins un solvant ;
- au moins un réservoir (1, 1') de stockage sous forte pression de ladite solution de chaque composé ;
- au moins un dispositif de mise sous pression pouvant aller de 3 à 300 bar ;
- au moins un dispositif de chauffage de la solution à une température supérieure au point d'ébullition du solvant ou à une température supérieure au point d'ébullition du mélange de solvants ;
▪ une chambre d'atomisation comprenant
- au moins un dispositif de dispersion de chaque solution sous un angle allant de 30 à 150° et à une pression allant de 0,0001 à 2 bar, les dispositifs de dispersion étant deux buses à cône creux (3) installées côte à côte dans la chambre d'atomisation, chacune équipée d'un système de chauffage électrique, et orientées l'une par rapport à l'autre de manière à ce que leurs jets s'interpénètrent ;
- au moins un dispositif de séparation de solvant ;
▪ un ou plusieurs dispositifs de récupération des nanoparticules de co-cristaux des composés choisis parmi un séparateur électrostatique, un cyclone (5), un cyclone comprenant un dispositif électrostatique.

16. Dispositif selon la revendication 15, dans lequel les buses sont deux buses (3) chauffées intégrées, et la chambre d'atomisation comprend deux cyclones axiaux (5) montés en parallèle et permettant une production semi-continue, et une pompe à vide (6).

17. Dispositif selon la revendication 16, dans lequel la pompe à vide (6) assure un écoulement permanent dans l'installation et permet d'extraire les vapeurs de solvant du dispositif.

## Patentansprüche

1. Verfahren zur Vorbereitung eines Co-Kristalls aus mindestens zwei Verbindungen, die durch Wasserstoffverbindungen, lonenverbindungen, Stapelverbindungen (π-π stacking) oder Van der Walls-Bindungen verbunden sind, umfassend die folgenden aufeinanderfolgenden Schritte:
▪ Vorbereitung von mindestens zwei Lösungen, die jeweils mindestens ein Lösungsmittel und mindestens eine organische, mineralische oder organometallische Verbindung umfassen, wobei sich diese Verbindungen durch Wasserstoffverbindungen, durch lonenverbindungen, durch Stapelverbindungen (π-π stacking) oder durch Van der Walls-Bindungen binden können;
▪ Erhitzung von Lösungen unter einem Druck von 3 bis 300 bar auf eine Temperatur über dem Siedepunkt des Lösungsmittels oder über dem Siedepunkt der Lösungsmittelmischung;
▪ Zerstäubung jeder Lösung in einer Zerstäuberkammer mittels mindestens einer Dispergiervorrichtung und unter einem Winkel von 30 bis 150° bei einem Druck von 0,0001 bis 2 bar, wobei die Dispergiervorrichtungen zwei Hohlkegeldüsen (3) sind, die nebeneinander in der Zerstäuberkammer installiert sind, jeweils mit einem elektrischen Heizsystem ausgestattet und so zueinander ausgerichtet sind, dass ihre Strahlen sich durchdringen;
▪ Trennung von Lösemitteln in gasförmiger Form.

2. Verfahren nach Anspruch 1, wobei die mindestens zwei Lösungen in mindestens zwei Reaktoren erhitzt werden, wobei die Reaktoren eine Zufuhr einer Lösung jeder Verbindung, mindestens eine Druckbeaufschlagungsvorrichtung, die von 3 bis 300 bar reichen kann, und mindestens eine Heizvorrichtung umfassen, und wobei die mindestens zwei Lösungen in der gleichen Zerstäuberkammer zerstäubt werden.

3. Verfahren nach Anspruch 1 oder 2, umfassend die Zubereitung von mindestens einer Lösung, umfassend
▪ zwei bis zehn Verbindungen; oder
▪ zwei Verbindungen; oder
▪ zwei Verbindungen in einem Molverhältnis ausgewählt aus 1/4, 1/3, 1/2, 1/1, 2/1, 3/1, 4/1; oder
▪ drei Verbindungen oder
▪ drei Verbindungen in einem Molverhältnis X/Y/Z, wobei X, Y und Z, gleich oder unterschiedlich, 1, 2, 3 oder 4 darstellen;
▪ vier Verbindungen; oder
▪ vier Verbindungen in einem Molverhältnis W/X/Y/Z, wobei W, X, Y und Z, gleich oder unterschiedlich, 1, 2, 3 oder 4 darstellen;
▪ fünf Verbindungen oder
▪ fünf Verbindungen in einem Molverhältnis V/W/X/Y/Z, wobei V, W, X, Y und Z, identisch oder unterschiedlich sind, für 1, 2, 3 oder 4 darstellen.

4. Verfahren nach einem der Ansprüche 1 bis 3, bei dem das Co-Kristall
▪ eine Mikrometergröße aufweist oder
▪ mindestens eine Größe von weniger als 500 µm aufweist; oder
▪ mindestens eine Größe von weniger als 100 µm aufweist oder
▪ eine submikrometrische Größe aufweist oder
▪ mindestens eine Größe zwischen 100 nm und 1000 nm aufweist; oder
▪ eine Nanometergröße aufweist; oder
▪ mindestens eine Größe von weniger als 100 nm aufweist oder
▪ eine Größe von 2 bis 100 nm aufweist oder
▪ eine Größe zwischen 5 und 90 nm aufweist oder
▪ eine Größe von 10 bis 80 nm aufweist oder
▪ eine Größe von 50 bis 300 nm aufweist oder
▪ eine Größe von 50 bis 200 nm aufweist oder
▪ eine Größe zwischen 50 und 120 nm aufweist oder
▪ eine Größe von 10 bis 100 nm aufweist oder
▪ eine Größe von 60 bis 100 nm aufweist.

5. Verfahren nach einem der Ansprüche 1 bis 4, das die Zubereitung von mindestens einer Lösung umfasst, die mindestens zwei organische, mineralische oder organometallische Verbindungen und mindestens zwei Lösungsmittel oder mindestens ein Lösungsmittel und mindestens ein Co-Lösungsmittel oder mindestens ein Lösungsmittel und mindestens ein Anti-Lösungsmittel einer der Verbindungen umfasst.

6. Verfahren nach einem der Ansprüche 1 bis 5, das einen letzten Schritt der Rückgewinnung der Co-Kristalle von Verbindungen mittels einer oder mehrerer Vorrichtungen umfasst, die unter einem elektrostatischen Separator, einem Zyklon (5), einem Zyklon, der eine elektrostatische Vorrichtung umfasst, ausgewählt sind.

7. Verfahren nach einem der Ansprüche 1 bis 6, das kontinuierlich oder halbkontinuierlich ist.

8. Verfahren nach einem der Ansprüche 1 bis 7, bei dem der Siedepunkt des Lösungsmittels oder der Lösungsmittelmischung unter 80 °C oder unter 60 °C liegt.

9. Verfahren nach einem der Ansprüche 1 bis 7, bei dem die Erhitzung der Lösungen unter einem Druck von 5 bis 150 bar oder von 10 bis 60 bar erfolgt.

10. Verfahren nach einem der Ansprüche 1 bis 9, bei dem die Erwärmung der Lösungen unter Druck eines Inertgases durchgeführt wird, das unter Stickstoff, Argon, Helium, Neon, Xenon ausgewählt ist.

11. Verfahren nach einem der Ansprüche 1 bis 9, wobei die Zerstäubung der Lösungen bei einem Druck von 0,001 bis 2 bar erfolgt.

12. Verfahren nach einem der Ansprüche 1 bis 11, wobei die Verbindungen unter Energieverbindungen, pharmazeutischen Verbindungen, phytopharmazeutischen Verbindungen, färbenden Verbindungen, Pigmenten, Tinten, Farben, Metalloxiden, ausgewählt werden.

13. Verfahren nach einem der Ansprüche 1 bis 12, wobei das Lösungsmittel aus Alkanen, beispielsweise Pentan (PE= 36 °C) oder Hexan (PE= 68 °C); Alkohole, beispielsweise Methanol (PE= 65 °C) oder Ethanol (PE= 78-79 °C); Thiole, beispielsweise Ethan-Thiol (PE= 35 °C); Aldehyde, beispielsweise Ethan (PE= 20 °C) oder Propionaldehyd (PE= 48 °C); Ketone, beispielsweise Aceton (PE= 56 °C); Ether, beispielsweise Methyltertbutylether (PE= 55 °C) oder Tetrahydrofuran (PE= 66 °C); Säureester, insbesondere Ameisensäureester, beispielsweise Methylformat (PE= 32 °C), Essigsäureester, beispielsweise Methylacetat (PE= 57-58 °C); Amine, beispielsweise Trimethylamin (PE= 2-3 °C), ausgewählt ist.

14. Verfahren nach einem der Ansprüche 1 bis 13, **dadurch gekennzeichnet, dass** die Lösungen in einer Gruppe von zwei Speicherbehältern (1, 1') unter hohem Druck gelagert werden, wobei die Lösungen in der Zerstäuberkammer zerstäubt werden, die zwei integrierte beheizte Hohlkegeldüsen (3) umfasst, und die Co-Kristalle in zwei parallel geschalteten Axialzyklone (5) zurückgewonnen werden, die eine halbkontinuierliche Produktion ermöglichen, und wobei eine Vakuumpumpe (6) für einen kontinuierlichen Durchfluss durch die Anlage sorgt und die Absaugung von Lösungsmitteldämpfen ermöglicht.

15. Vorrichtung zur Durchführung eines Verfahrens nach den Ansprüchen 1 bis 14, umfassend
▪ mindestens zwei Reaktoren mit jeweils
- Zuführen einer Lösung jeder Verbindung und mindestens eines Lösungsmittels;
- mindestens einen Behälter (1, 1') zur Lagerung unter hohem Druck der Lösung jeder Verbindung;
- mindestens eine Druckbeaufschlagungsvorrichtung mit einem Druckbereich von 3 bis 300 bar;
- mindestens eine Vorrichtung zur Erhitzung der Lösung bei einer Temperatur über dem Siedepunkt des Lösungsmittels oder bei einer Temperatur über dem Siedepunkt der Lösungsmittelmischung;
▪ eine Zerstäuberkammer umfassend
- mindestens eine Dispergiervorrichtung für jede Lösung in einem Winkel von 30 bis 150° und bei einem Druck von 0,0001 bis 2 bar, wobei die Dispergiervorrichtungen zwei Hohlkegeldüsen (3) sind, die nebeneinander in der Zerstäuberkammer installiert sind, jeweils mit einem elektrischen Heizsystem ausgestattet und so zueinander ausgerichtet sind, dass ihre Strahlen sich durchdringen;
- mindestens eine Lösungsmitteltrennvorrichtung;
▪ eine oder mehrere Vorrichtungen zur Rückgewinnung der Nanopartikel von Co-Kristallen der Verbindungen, ausgewählt aus einem elektrostatischen Separator, einem Zyklon (5), einem Zyklon umfassend eine elektrostatische Vorrichtung.

16. Vorrichtung nach Anspruch 15, wobei es sich bei den Düsen um zwei integrierte beheizte Düsen (3) handelt, und die Zerstäuberkammer zwei parallel montierte Axialzyklone (5) umfasst, die eine halbkontinuierliche Produktion ermöglichen, und eine Vakuumpumpe (6).

17. Vorrichtung nach Anspruch 16, wobei die Vakuumpumpe (6) einen ständigen Durchfluss in der Anlage sicherstellt und es ermöglicht, Lösungsmitteldämpfe aus der Vorrichtung abzusaugen.

## Claims

1. A method for preparing a co-crystal of at least two compounds bound through hydrogen bonds, ionic bonds, bonds of the stacking type (π- π stacking) or Van der Waals bonds, comprising the successive steps:
▪ preparation of at least two solutions, each comprising at least one solvent and at least one organic, mineral or organometal compound, these compounds may be bound through hydrogen bonds, ionic bonds, bonds of the stacking type (π- π stacking) or through Van der Waals bonds;
▪ heating the solutions, under a pressure ranging from 3 to 300 bars, at a temperature above the boiling point of the solvent or at a temperature above the boiling point of the mixture of solvents;
▪ atomization in a same atomization chamber of each solution by means of at least one dispersion device and under an angle ranging from 30 to 150° at a pressure ranging from 0.0001 to 2 bars, the dispersion devices being two hollow cone nozzles (3) installed side by side in the atomization chamber, each equipped with an electric heating system and oriented relatively to each other so that their jets interpenetrate each other;
▪ separation of the solvents in a gaseous form.

2. The method according to claim 1, for which these at least two solutions are heated in at least two reactors, these reactors comprising a supply of a solution of each compound, at least one pressurization device which may range from 3 to 300 bars and at least one heating device, and for which these at least two solutions are atomized in the same atomization chamber.

3. The method according to claim 1 or 2 comprising the preparation of at least one solution comprising
▪ two to ten compounds; or
▪ two compounds; or
▪ two compounds in a molar ratio selected from among 1/4, 1/3, 1/2, 1/1, 2/1, 3/1, 4/1; or
▪ three compounds; or
▪ three compounds in a molar ratio X/Y/Z wherein X, Y and Z, either identical or different, represent 1, 2, 3 or 4;
▪ four compounds; or
▪ four compounds in a molar ratio W/X/Y/Z wherein W, X, Y and Z, either identical or different, represent 1, 2, 3 or 4;
▪ five compounds; or
▪ five compounds in a molar ratio V/W/X/Y/Z wherein V, W, X, Y and Z, either identical or different, represent 1, 2, 3 or 4.

4. A method according to one of claims 1 to 3 for which the co-crystal
▪ is of a micrometric size; or
▪ has at least one dimension of less than 500 µm; or
▪ has at least one dimension of less than 100 µm; or
▪ is of submicrometric size; or
▪ has at least one dimension comprised between 100 nm and 1,000 nm; or
▪ is of a nanometric size; or
▪ has at least one dimension of less than 100 nm; or
▪ is of a size ranging from 2 to 100 nm; or
▪ is of a size ranging from 5 to 90 nm; or
▪ is of a size ranging from 10 to 80 nm; or
▪ is of a size ranging from 50 to 300 nm; or
▪ is of a size ranging from 50 to 200 nm; or
▪ is of a size ranging from 50 to 120 nm; or
▪ is of a size ranging from 10 to 100 nm; or
▪ is of a size ranging from 60 to 100 nm.

5. The method according to one of claims 1 to 4, comprising the preparation of at least one solution comprising at least two organic, mineral or organometal compounds and at least two solvents or at least one solvent and at least one co-solvent or at least one solvent and at least one anti-solvent of one of the compounds.

6. The method according to one of claims 1 to 5, comprising a final step for recovering co-crystals of compounds by means of one or several devices selected from among an electrostatic separator, a cyclone (5), a cyclone comprising an electrostatic device.

7. The method according to one of claims 1 to 6 which is continuous or semi-continuous.

8. The method according to one of claims 1 to 7, for which the boiling point of the solvent or of the mixture of solvents is less than 80°C or is less than 60°C.

9. The method according to one of claims 1 to 7, for which the heating of the solutions is carried out under a pressure ranging from 5 to 150 bars or ranging from 10 to 60 bars.

10. The method according to one of claims 1 to 9, for which the heating of the solutions is carried out under the pressure of an inert gas selected from among nitrogen, argon, helium, neon, xenon.

11. The method according to one of claims 1 to 9, for which the atomization of the solutions is carried out at a pressure ranging from 0.001 to 2 bars.

12. The method according to one of claims 1 to 11, for which the compounds are selected from among energy compounds, pharmaceutical compounds, phytopharmaceutical compounds, coloring compounds, pigments, inks, paints, metal oxides.

13. The method according to one of claims 1 to 12, for which the solvent is selected from among alkanes, for example pentane (BP = 36°C) or hexane (BP = 68°C); alcohols, for example methanol (BP = 65°C) or ethanol (BP = 78-79°C); thiols, for example ethane-thiol (BP = 35°C); aldehydes, for example ethanal (BP = 20°C) or propionic aldehyde (BP = 48°C); ketones, for example acetone (BP = 56°C); ethers, for example methyl-tert-butyl ether (BP = 55°C) or tetrahydrofurane (BP = 66°C); acid esters, notably esters of formic acid, for example methyl formiate (BP = 32°C), acetic acid esters, for example methyl acetate (BP = 57-58°C); amines, for example trimethylamine (BP = 2-3° C).

14. The method according to one of claims 1 to 13, **characterized in that** these solutions are stored in a set of two tanks (1, 1') for storing under a strong pressure, these solutions being atomized in the atomization chamber comprising two integrated heated hollow cone nozzles (3), and said co-crystals are recovered in two axial cyclones (5) mounted in parallel and allowing a semi-continuous production, and wherein a vacuum pump (6) ensures a permanent flow in the installation and allows extraction of the solvent vapors.

15. A device allowing the implementation of a method according to claims 1 to 14, comprising
▪ at least two reactors, each comprising
- a supply of a solution of each compound and of at least one solvent;
- at least one tank (1, 1') for storing under a strong pressure said solution of each compound;
- at least one pressurization device which may range from 3 to 300 bars;
- at least one device for heating the solution at a temperature above the boiling point of the solvent or at a temperature above the boiling point of the mixture of solvents;
▪ an atomization chamber comprising
- at least one dispersion device for each solution under an angle ranging from 30 to 150° and at a pressure ranging from 0.0001 to 2 bars, the dispersion devices being two hollow cone nozzles (3) installed side by side in the atomization chamber, each equipped with an electric heating system and oriented relatively to each other so that their jets interpenetrate each other;
- at least one solvent separation device;
▪ one or several devices for recovering nanoparticles of compounds, selected from among an electrostatic separator, a cyclone (5), a cyclone comprising an electrostatic device.

16. The device according to claim 15, in which the nozzles are two integrated heated nozzles (3), and the atomization chamber comprises two axial cyclones (5) mounted in parallel and allowing a semi-continuous production, and a vacuum pump (6).

17. The device according to claim 16, in which the vacuum pump (6) ensures a permanent flow in the installation and allows extraction of the solvent vapors from the device.
